# EUROPEAN PATENT APPLICATION

(11) **EP 3 842 089 A1**
(43) Date of publication of application: **30.06.2021**
(21) Application number: 20214259.2
(22) Date of filing: 15.12.2020
(51) Int. Cl.: A61M 5/315, A61M 5/142, A61M 5/168, A61M 5/20, A61M 5/30

(54) **ADMINISTRATION DEVICE**

(30) Priority: 23.12.2019 IT 201900025345
(71) Applicant: Medirio SA, 3930 Visp (CH)
(72) Inventor: CALASSO, Irio Giuseppe, 3930 Visp (CH); DE DONATIS, Matteo, 3930 Visp (CH)
(74) Representative: Mitola, Marco

(57) **Abstract**

A medical device and system for the administration of a medical substance or for dosing body fluids comprising a rotor, rotating around an axis X-X, operatively connected to an injection device of said medical substance, a rotor locking/unlocking device mechanically separate from the rotor and integral in rotation therewith, wherein the locking/unlocking device comprises, in one piece, a disc, centered with respect to said axis X-X, from which a plurality of flexible fins radially unravel. The flexible fins are configured to flex around transverse hinges at a connection portion with the disc, and are angularly spaced so as to define gaps between adjacent pairs of fins which prevent interferences between adjacent fins in the respective flexing movement. The flexible fins are associated with ferromagnetic means for the operation of the same fins and locking/unlocking means of the rotation of the disc. The disc and fins are obtained from a single plastic body.

## Description

### FIELD OF APPLICATION

The present invention relates to a device and a medical system for the administration of a medical substance or for dosing body fluids and the related manufacturing method.

### PRIOR ART

As is known, some medical conditions require a regular dosage of a continuous infusion of medicines, others require timely or periodic administration. These medications are often provided as liquid solutions to be infused, e.g., transdermally, intramuscularly or intravenously. Diabetic patients, for example, may require insulin. In an attempt to facilitate the lives of these patients, various infusion devices have been developed.

The infusion devices known in the art typically comprise simple injection pen devices or complex pump devices, which use mechanical or electromechanical pumping to provide the medication to a patient through the skin.

The injection pen devices require the patient to repeatedly perform a new injection, are not discreet, and are associated with a feeling of discomfort, fear of injection, and pain. Furthermore, they often lack any type of control, feedback and safety features. However, they have the advantage of being inexpensive and relatively simple to use.

The pump devices instead comprise a series of elements required for operation and control, e.g., a processor, electrical components, a battery, buttons or switches located on the device housing, visual feedback via text or graphic display, etc.

Such known devices, whether of the pen or pump type, must ensure the administration or infusion of the correct predetermined dose in total safety. This means that on the one hand they must ensure precise control of the dose delivered and on the other they must avoid any uncontrolled or accidental delivery.

In particular, some pump devices are part of a delivery system comprising the actual delivery device, applied in direct contact with the patient's skin (also called a POD) and an actuation device (called a 'handheld') of the delivery device. Some additional pump solutions include a delivery device connected via tubes to a needle which infuses the medication into the patient. The POD-type delivery device is applied fixed to the skin, usually by adhesive, until the medical fluid housed in a special reservoir associated with the same device is completely exhausted.

A proprietary solution of the holder of the present patent applications includes that the actuation device is instead a portable device which, if required, is gripped by the user and brought close to the delivery device (a few millimeters away). The actuation device contains magnets which, applied at a close distance to the delivery device, activate the respective delivery means. In particular, the delivery device comprises a rotor which rotates by magnetic coupling and is operatively connected to a pump in turn connected, in input, to the reservoir of medical substance to be delivered and, in output, to a delivery needle which penetrates the patient's skin. In a possible embodiment, the delivery means are normally locked by special locking devices which prevent any accidental and unintentional delivery of the medical substance. In another possible embodiment, the magnets of the actuation device, if placed at a close distance with respect to the delivery device, are capable of moving or unlocking the rotor locking devices so as to allow the rotation of the rotor and the delivery of medical substance thereto.

A critical element of the system consists precisely of the delivery device and, in particular, of the locking device contained therein.

In fact, the locking device must ensure the constancy, i.e., the repeatability of the unlocking force applied from the outside by the application device.

In fact, if the unlocking force is not calibrated, therefore repeatable, the system could lead to false actuations and/or failed actuations. In addition, the difficult repeatability of the locking/unlocking operations is also given by the variable of the distance which the user interposes between the actuation device and the delivery device.

In order to ensure the required repeatability of the system (and therefore constancy of the locking/unlocking force) and also in order to ensure an allowed actuation radius, various solutions have been implemented in the art which have the disadvantage of entailing considerable manufacturing costs; nevertheless, the known solutions do not always manage to ensure such constancy of the locking/unlocking force of the rotor.

### PRESENTATION OF THE INVENTION

Therefore, the need is felt to solve the drawbacks and limitations mentioned above with reference to the prior art.

Such a need is satisfied by a medical device in accordance with claim 1, a medical system in accordance with claim 16 and a manufacturing method of a medical device in accordance with claim 17.

### DESCRIPTION OF THE DRAWINGS

Further features and advantages of the present invention will become more comprehensible from the following description of preferred embodiments thereof given by way of non-limiting examples, in which:

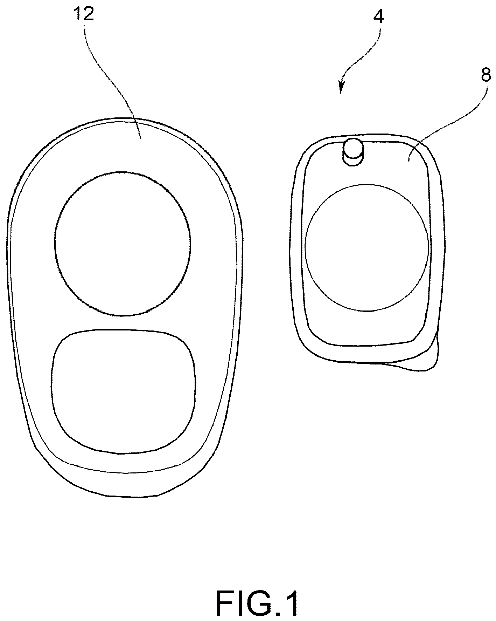

figure 1 shows a perspective view of a delivery system comprising a delivery device and an actuation device of the delivery device, in accordance with an embodiment of the present invention;

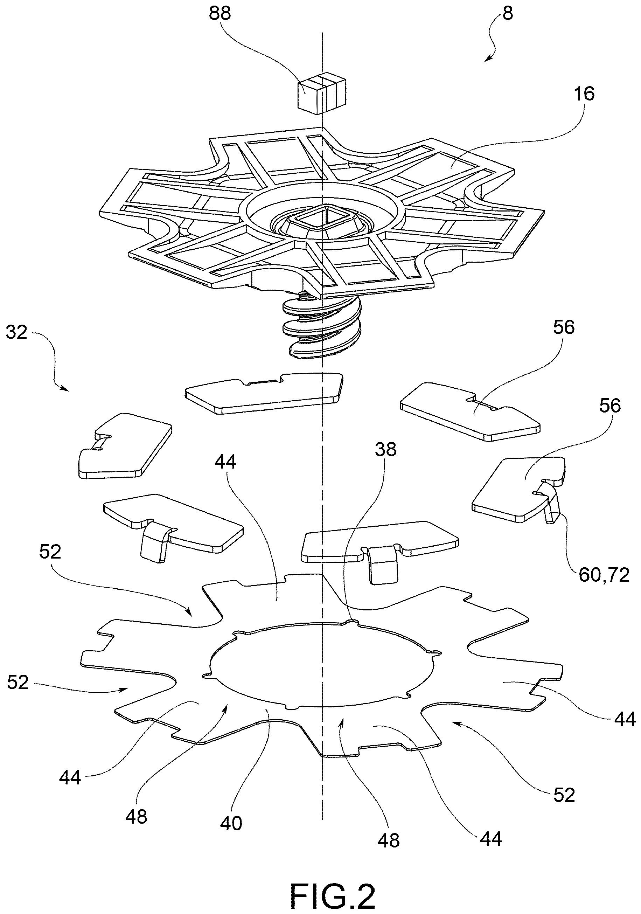

figure 2 shows a perspective view, in separate parts, of a delivery device in accordance with an embodiment of the present invention;

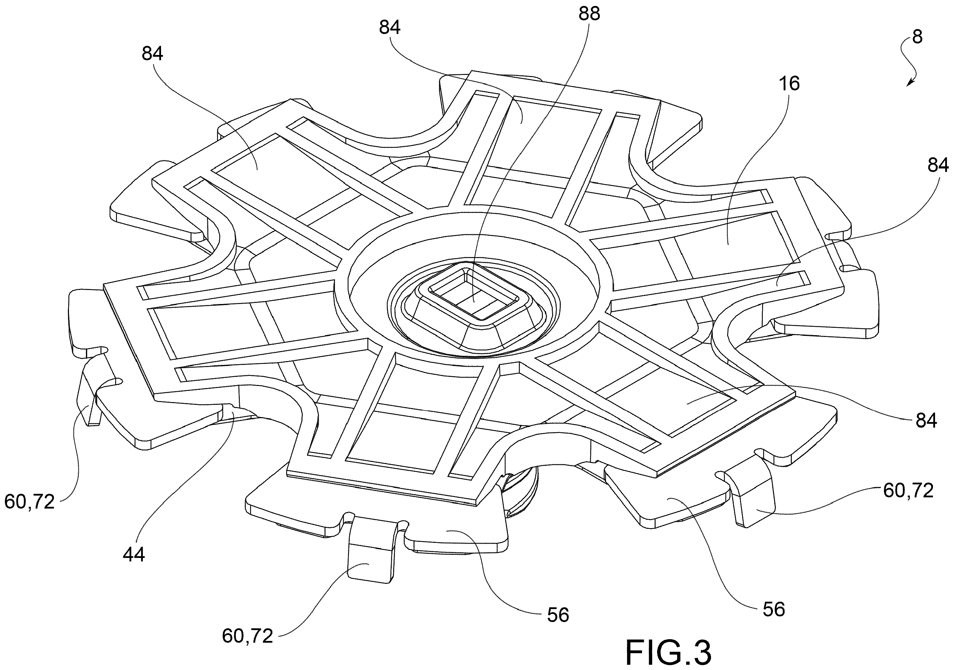

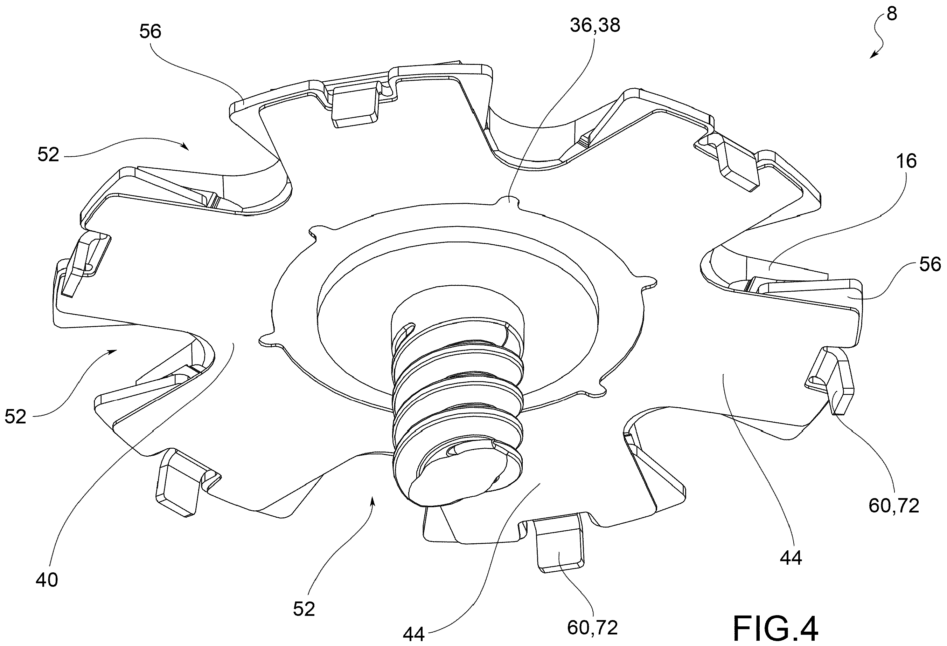

figures 3-4 show perspective views, from different angles, of the delivery device of figure 2, in an assembled configuration.

Elements or parts in common to the embodiments described will be indicated hereafter using the same reference numerals.

### DETAILED DESCRIPTION

With reference to the aforesaid figures, a system of transdermal, intramuscular, subcutaneous or intravenous administration of a medical substance has been indicated as a whole with 4.

As mentioned above, the delivery system 4 comprises a transdermal, intramuscular, subcutaneous or intravenous delivery device 8 (said 'POD'), which in use is applied to the patient's skin or implanted in the body, and an actuation device 12 (said 'handheld') of said delivery device 8 which if required is gripped by the user and brought close to the delivery device 8 so as to activate the delivery step of the medical substance, as better described below.

More in detail, a medical device according to the present invention is a device which is adapted to perform medical treatments when put in contact with a patient and when it externally receives energy from the portable device by temporarily positioning the portable device close to the POD, when a medical treatment is required. "In contact" means both in cutaneous contact with the user, e.g., removably fixed, e.g., by an adhesive base, on the patient's skin, or more generally in contact with the body, comprising a device inside the body, fixed at least in part in a body cavity or implanted in the body.

According to an embodiment, the medical device is an implanted device or a device partially inside the body and partially outside the body, e.g., a catheter, adapted to regulate the flow of a fluid. The medical device may then be obtained as a valve device adapted to enable/disable fluid flow or vary the flow rate of a fluid, e.g., a body fluid, or as a continuous delivery device, adapted to deliver a continuous flow of a medication with a variable flow rate for an extended period of time. The medical treatment may therefore result from regulating the flow of a fluid from the outside towards the body or from the inside towards the outside, e.g. draining excess fluid produced by the body, e.g., due to a pathological condition or regulating the flow of a body fluid inside the body, i.e., from one part of the body to another.

The intramuscular, subcutaneous or intravenous transdermal delivery device 8 comprises a rotor 16, rotating around an axis of rotation X-X, operatively connected to an injection device 20 of said medical substance equipped with a needle 24 and a reservoir 28 of medical substance. For the purposes of the present invention, the type, shape and size of the injection device 20, needle 24 and reservoir 28 or pumping instrument are not relevant or limiting.

The delivery device 8 comprises a locking/unlocking device 32 of the rotor 16 mechanically separate from the rotor 16 and integral in rotation with the rotor 16 around said axis of rotation X-X. The coupling between the rotor 16 and the locking/unlocking device is preferably a shape coupling, obtained for example by keys or tabs 36 which engage in respective seats 38.

The locking/unlocking device 32 comprises, in one piece, a disc 40, centered (i.e., axially symmetrical) with respect to said axis of rotation X-X, from which a plurality of flexible fins 44 radially unravel.

It should be noted that the flexible fins 44 are elastically flexible.

In other words, the flexing movement is reversible and the fins do not have plastic or permanent deformations as a result of the flexing movement thereof. Thereby the flexible fins 44 can elastically flex, repeatedly, during all the respective actuations requested by the user, always ensuring the same behavior and the same elastic resistance.

The flexible fins 44 are configured to flex around hinges 48, e.g., transverse, at a connection portion with the disc 40.

Preferably, the flexible fins 44 are substantially coplanar with the disc 40, at least in a non-deformed or unflexed configuration.

The hinges 48 are hinges lying on a plane perpendicular to the axis of rotation X-X. Preferably the hinges 48 are arranged, on such a plane perpendicular to the axis of rotation X-X, along tangential directions with respect to concentric circumferences with said axis of rotation X-X. Thereby, the flexible fins 44 can easily elastically flex, around the hinges 48, in substantially radial directions, passing through said axis of rotation X-X.

The flexible fins 44 are preferably equally angularly spaced from each other so as to define gaps 52 between adjacent pairs of flexible fins 44 which prevent interferences between adjacent fins in the respective flexing movement around said hinges 48.

The presence of said gaps 52 prevents that during the elastic flexing movement the flexible fins 44, adjacent to each other, can also accidentally interfere and/or reciprocally overlap each other.

The flexible fins 44 are associated with ferromagnetic means 56 for the operation of the same fins and locking/unlocking means 60 for the rotation of the disc 40 and therefore also the rotor 16, integral in rotation with the locking/unlocking device 32. It should be noted that the term ferromagnetic means is intended as means having magnetic or ferromagnetic properties.

The disc 40 and/or the flexible fins 44 are preferably obtained from a single body made of plastic material. Plastic material is intended as any material of polymeric origin, possibly reinforced or loaded in one or more different layers.

There are several modes for obtaining the locking/unlocking device 32.

According to a possible embodiment, said single body is a die-cut of a plastic sheet of constant thickness. The main advantage of this solution is that the constant thickness of the sheet is easily reproducible and controllable; furthermore, the production by die-cutting is also easily repeatable and reproducible. Therefore, with such a solution it is easy to obtain fins which have a constant and repeatable elasticity over time.

According to possible embodiments, the die-cut is associated with the rotor 16 by pins, staples, nails or even by interlocking insertion inside a sandwich structure.

According to possible embodiments, the die-cut is associated with the rotor 16 by gluing. According to a possible embodiment, the die-cut has a layer of glue, after die-cutting.

As mentioned above, the locking/unlocking device is provided with ferromagnetic means 56 for controlling the movement of the flexible fins 44 to which said ferromagnetic means 56 are constrained.

The ferromagnetic means typically comprise flat bases or plates in ferromagnetic material. Said bases or plates are at least partially counter-shaped with respect to the corresponding flexible fins 44 to which they are applied.

According to a possible embodiment, the flexible fins 44 are provided with a layer of glue to which the ferromagnetic means 56 are constrained by gluing. The use of a layer of glue is particularly advantageous in the solution in which the single body is obtained by die-cutting. In fact, in this case it is possible to provide the sheet of material from which the die-cut will then be obtained with glue in advance. This does not exclude that the use of adhesives is also applicable to solutions in which the single body is obtained by injection molding.

The locking/unlocking means 60 must be able to allow and/or inhibit the rotation of the rotor 16.

According to a possible embodiment, the locking/unlocking means 60 comprise at least one tooth 72 in relief. In a rest or locked condition, the tooth 72 is housed in a related recess 74 so as to obtain a stop or lock on the rotation of the rotor 16.

For example, the locking/unlocking means 60 are integral and/or in one piece with the flexible fins 44.

It is also possible to include the locking/unlocking means 60 integral and/or in one piece with the ferromagnetic means 56 associated with the flexible fins 44. For example, this solution is particularly suitable for the use of a die-cut obtained from a sheet.

The hinges 48 can be made in various manners.

According to a possible embodiment, said hinges 48 comprise, at each flexible fin 44, a monobloc having a circular sector conformation, which follows the conformation of the disc 40.

As already described, the locking/unlocking device 32 is integral in rotation with the rotor 16. In particular, the rotor 16, according to one embodiment, comprises a plurality of radial fingers 84 (with respect to the same axis of rotation X-X) and at least partially superposed to the flexible fins 44 and/or the ferromagnetic means 56 so as to obtain an end-stop to the flexing movement of the flexible fins 44.

In other words, the flexible fins 44 under the thrust of the ferromagnetic means 56 can rise, i.e., flex towards the associable rotor 16 until abutting against the radial fingers 84 which act as the end-stop. Upon the cessation of the thrust action of the ferromagnetic means 56 the fins return to the initial or non-deformed or rest position.

At least one measuring magnet 16 is associated with the rotor 88 which is adapted to allow the measurement of the actual rotation performed by the rotor 16 following the unlocking in rotation thereof.

Due to this measurement, the delivery system 4 is capable of monitoring exactly the amount of fluid delivered (which is a function of the number of revolutions and/or fractions of revolutions performed by the rotor 16).

Preferably, said measuring magnet 88 is located at an inner radius of the rotor 16, lower with respect to the radial extension of the flexible fins 44.

According to an embodiment, said measuring magnet 88 is mounted coaxially to the axis of rotation X-X of the rotor 16 and can have at least one non-axial-symmetric portion with respect to said axis of rotation X-X. The non-axial-symmetric portion serves to obtain or enlarge a 'sensitive' variation of magnetic flux which can be transposed in counting the rotor revolutions 16.

As mentioned, the delivery system 4 comprises both the delivery device 8 and the actuation device 12.

The actuation device 12 is configured so as to interface with the corresponding delivery device 8. To this end, the actuation device 12 comprises at least one actuation magnet (not shown) adapted to move the flexible fins 44 of the delivery device 8, by means of the respective ferromagnetic means 56, so as to allow the unlocking and subsequent locking of the rotor 16 to allow/inhibit the delivery of medical substance, respectively.

Furthermore, the actuation device 12 comprises at least one magnetic element which by rotating induces the rotation of the rotor in the delivery device by magnetic coupling.

Furthermore, the actuation device 12 keeps track of the number of revolutions performed by the rotor 16 so as to monitor the actual amount of substance injected; the actuation device 12 also allows to set the amount of substance to be injected so as to actuate the delivery device until the set amount has been reached.

The manufacturing method of a delivery device in accordance with the present invention will now be described.

In particular, the manufacturing method of a transdermal, intramuscular or intravenous delivery device 8 of a medical substance, comprises the steps of:

preparing a rotor 16, rotating around an axis of rotation X-X, operatively connected to an injection device 20 of said medical substance equipped with a needle 24 and a reservoir 28 of medical substance,

preparing a locking/unlocking device 32 of the rotor 16 mechanically separate from the rotor 16 and integral in rotation with the rotor 16 around said axis of rotation X-X, in which

the locking/unlocking device 32 comprises, in one piece, a disc 40, centered with respect to said axis of rotation X-X, from which a plurality of flexible fins 44 radially unravel, configured to flex around hinges 48 at a connection portion with the disc 40, said flexible fins 44 being angularly spaced from each other so as to define gaps 52 between adjacent pairs of flexible fins 44, where said gaps 52 prevent interferences between adjacent fins in the respective flexing movement around the hinges 48,
associating the flexible fins 44 with ferromagnetic means 56 for the operation of the same flexible fins 44 and locking/unlocking means 60 for the rotation of the disc 40 and, with the latter, the rotor 16,
wherein the disc 40 and the flexible fins 44 are obtained from a single plastic body.

In particular, the step of making the single body can be included by means of a single die-cut of a plastic sheet of constant thickness; preferably, said die-cut comprises a layer of adhesive for the subsequent gluing of the ferromagnetic means 56 and for possible gluing to the rotor.

The operation of a system and a delivery device in accordance with the present invention will now be described.

In particular, when the user must perform an injection of medical substance, he/she brings the actuation device 12 (hand-held) close to the delivery device 8 applied to the user.

After selecting the dose to be delivered, the actuation device is capable of unlocking the rotor 16. In particular, in a rest or non-delivery condition, the rotor 16 is locked in rotation due to the fact that the flexible fins 44 are in a rest or lowered condition so that the respective teeth 72 are constrained in related recesses.

When the actuation magnets of the actuation device 12 are placed close to the delivery device 8, said actuation magnets lift, attracting them thereto, the ferromagnetic means 56 of the locking/unlocking device 32 which, in turn, drag the respective flexible fins 44 therewith in the upwards movement. The flexible fins 44 can flex, substantially radially, by pivoting on the respective hinges 48. Thereby the teeth 72 are released from the respective recesses 74, therefore releasing the rotor from any locking of the rotation around the axis of rotation X-X. The lifting movement of the flexible fins is stopped by the stop between the fins themselves and the stops 64 obtained on the rotor 16.

The rotor 16 may then actuate the injection device 20 to which it is operatively connected, in a known manner. During the delivery of the medical liquid, the measuring magnet 88 measures the number of revolutions (and/or fractions of revolutions) performed by the rotor 16 and stops the delivery upon reaching the preset amount of medical substance. Then, when the delivery ceases with the removal of the control device from that of delivery, the actuation magnets cease to influence the elastic fins 44 which can elastically return to the rest position thereof so as to return the teeth 72 to the respective seats 68, preventing any involuntary rotation of the rotor 16 and therefore avoiding any uncontrolled delivery of medical liquid.

As can be appreciated from the description above, the present invention makes it possible to overcome the drawbacks presented in the prior art.

In particular, the present invention makes it possible to obtain a locking/unlocking device which can be produced in scale so as to present repeatable physical and geometric features: in other words, the locking/unlocking force of the rotor of the delivery device is controlled and constant in the serial production of the same device.

At the same time, the device is simple and economical to create and assemble.

The calibration of the locking/unlocking force allows to prevent both failed actuations and accidental actuations (the latter extremely dangerous) of the delivery means of the medical substance.

A person skilled in the art may make several changes and adjustments to the devices, systems and methods described above in order to meet specific and incidental needs, all falling within the scope of protection defined in the following claims.

## Claims

1. Medical device (8) for the administration of a medical substance or for dosing body fluids comprising:
- a rotor (16), rotating around an axis of rotation (X-X), operatively connected to an injection device (20) of said medical substance equipped with a needle (24) and a reservoir (28) of medical substance,
- a locking/unlocking device (32) of the rotor (16) separate from the rotor (16) and integral in rotation with the rotor (16) around said axis of rotation (X-X), with
- the locking/unlocking device (32) comprises a disc (40), centred with respect to said axis of rotation (X-X), from which a plurality of flexible fins radially unravel (44),
- wherein the flexible fins (44) are configured to flex around hinges (48) at a connection portion with the disc (40),
- wherein the flexible fins (44) are angularly spaced so as to define gaps (52) between adjacent pairs of flexible fins (44) which prevent the interference between adjacent fins (44) in their respective flexing movement around the hinges (48),
- wherein the flexible fins (44) are associated with ferromagnetic means (56) for the operation of said flexible fins (44) and locking/unlocking means (60) of the rotation of the disc (40) and the rotor (16),
- wherein the disc (40) and/or the flexible fins (44) are obtained from a single body such as a die-cut of a plastic sheet.

2. The medical device (8) according to claim 1, wherein said flexible fins (44) are substantially coplanar with the disc (40).

3. The medical device (8) according to claim 1 or 2, wherein said single body is a die-cut of a plastic sheet of constant thickness.

4. The medical device (8) according to any of the preceding claims, wherein a layer of glue is present between the flexible fins (44) and the ferromagnetic means (56).

5. The medical device (8) according to any of the preceding claims, wherein a layer of glue is applied in advance to the plastic sheet from which the die-cut is made.

6. The medical device (8) according to any of the claims from 1 to 5, wherein the die-cut, comprising the flexible fins (44), is stuck or glued to the rotor (16).

7. The medical device (8) according to claim 6, wherein the rotor (16) has pins for the relative positioning of the die-cut to said rotor.

8. The medical device (8) according to any of the claims from 1 to 7, wherein the locking/unlocking means (60) comprise at least one tooth (72) in relief.

9. The medical device (8) according to any of the claims from 1 to 8, wherein said locking/unlocking means (60) are integral with and/or in one piece with the flexible fins (44).

10. The medical device (8) according to any of the claims from 1 to 9, wherein said locking/unlocking means (60) are integral and/or in one piece with the ferromagnetic means (56) associated with the flexible fins (44).

11. The medical device (8) according to any of the claims from 1 to 10, wherein said hinges (48) comprise, at each flexible fin (44), a monobloc having a circular sector conformation, which follows the conformation of the disc (40) .

12. The medical device (8) according to any of the claims from 1 to 11, wherein the rotor (16) comprises a plurality of radial fingers (84) at least partially superposed to the flexible fins (44) and/or ferromagnetic means (56) so as to realise an end-stop to the flexing movement of the flexible fins (44).

13. The medical device (8) according to any of the preceding claims, wherein at least one suitable measuring magnet (88) is associated with the rotor (16) suitable to allow the measurement of the actual rotation performed by the rotor (16) following its release in rotation.

14. The medical device (8) according to claim 13, wherein said measuring magnet (88) is placed coaxially to the rotor (16).

15. The medical device (8) according to any of the claims from 13 to 14, wherein said measuring magnet (88) is mounted coaxially to the axis of rotation (X-X) of the rotor (16) and has at least one non-axial-symmetric portion with respect to said axis of rotation (X-X).

16. A medical system (4) for the administration of a medical substance or for dosing body fluids comprising a medical device (8) according to any of the preceding claims and an actuation device (12) of said medical device (4) wherein the actuation device (12) comprises:
- at least one actuation magnet suitable to move the flexible fins (44) of the delivery device (8), by means of the respective ferromagnetic means (56), so as to allow the unlocking and subsequent locking of the rotor (16) to respectively allow/inhibit the delivery of medical substance.

17. A method for the manufacture of a medical device (8) for the administration of a medical substance or for dosing body fluids, comprising the steps of:
- preparing a rotor (16), rotating around an axis of rotation (X-X), operatively connected to an injection device (20) of said medical substance equipped with a needle (24) and a reservoir (28) of medical substance,
- preparing a locking/unlocking device (32) of the rotor (16) mechanically separate from the rotor (16) and integral in rotation with the rotor (16) around said axis of rotation (X-X),
with
- the locking/unlocking device (32) comprises a disc (40), centred with respect to said axis of rotation (X-X), from which a plurality of flexible fins radially unravel (44) configured to flex around hinges (48) at a connection portion with the disc (40), said flexible fins (44) being angularly spaced so as to define gaps (52) between adjacent pairs of flexible fins (44) that prevent interference between adjacent flexible fins (44) in their respective flexing movement around said hinges (48),
- associating the flexible fins (44) with ferromagnetic means (56) for the operation of said flexible fins (44) and locking/unlocking means (60) for the rotation of the disc (40) and the rotor (16),
- wherein the disc (40) and/or the flexible fins (44) are obtained from a single body from a single die-cut of a plastic sheet.

18. The method according to claim 17, wherein the step of making said single body by means of a single die-cut of a sheet of plastic material of constant thickness is provided for.

19. The method according to claim 17, wherein the step of assembling the die-cut with the ferromagnetic elements (56) to the rotor (16) is provided for.

20. The method according to claim 17, 18 or 19, wherein this blank comprises a layer of adhesive for subsequent gluing of the ferromagnetic means (56).

21. The method according to claim 17, 18 or 19, wherein said die-cut comprises a layer of glue for subsequent gluing to the rotor (16).
